# EUROPEAN PATENT SPECIFICATION

(11) **EP 2 078 522 B1**
(45) Date of publication and mention of the grant of the patent: **05.09.2012**
(21) Application number: 07831097.6
(22) Date of filing: 26.10.2007
(51) Int. Cl.: A61K 31/191, A61P 27/14, A61P 37/08, A61K 31/19, A61K 9/00

(54) **COMPOSITION FOR TREATING ALLERGY**
ZUSAMMENSETZUNG ZUR BEHANDLUNG VON ALLERGIEN
COMPOSITION POUR LE TRAITEMENT DE L'ALLERGIE

(30) Priority: 30.10.2006 JP 2006294364
(43) Date of publication of application: 15.07.2009
(73) Proprietor: Ophtecs Corporation, Hyogo 6500047 (JP)
(72) Inventor: HISAMURA, Ryuji, Toyooka-shi Hyogo 6680831 (JP); NAKAMURA, Shigeru, Toyooka-shi Hyogo 6680831 (JP); YONEDA, Toyoaki, Kobe-shi Hyogo 6500047 (JP)
(74) Representative: Albrecht, Thomas
(86) International application number: PCT/JP2007/071363
(87) International publication number: WO 2008/053982

(56) References cited:
- EP-A1- 1 674 091
- JP-A- 2001 089 366
- JP-A- 2005 247 821
- JP-A- 2005 306 815
- MAEDA-YAMAMOTO MARI ET AL: "Effects and safety of consecutive intake of Benifuuki green tea and enhancement of the effect by ginger extract in subjects with Japanese cedar-pollinosis", NIPPON SHOKUHIN KAGAKU KOGAKU KAISHI, vol. 52, no. 12, 2005, pages 584-593, XP002612730, ISSN: 1341-027X
- HINGORANI ET AL: "Therapeutic options in ocular allergic disease", DRUGS, ADIS INTERNATIONAL LTD, NZ, vol. 50, no. 2, 1 January 1995 (1995-01-01), pages 208-221, XP002112535, ISSN: 0012-6667, DOI: DOI:10.2165/00003495-199550020-00002
- CHURCH M K ET AL: "Human ocular mast cells", CURRENT OPINION IN ALLERGY AND CLINICAL IMMUNOLOGY, LIPPINCOTT WILLIAMS AND WILKINS, US, vol. 2, no. 5, 1 October 2002 (2002-10-01) , pages 419-422, XP008130048, ISSN: 1528-4050

## Description

### TECHNICAL FIELD

The present invention relates to an antiallergenic composition comprising 3-hydroxybutyric acid and a salt thereof.

### BACKGROUND ART

The factors of causing an allergy include pollen, dust, ticks, molds and chemicals. Particularly, pollinosis has become a social problem in Japan, and it is reported that not less than 15 % of Japanese people are allergic to cedar pollen (2002 Edition of Nasal Allergy Diagnosis Guideline, p. 3).

Ocular itching is a typical symptom of pollinosis. The conjunctiva of an eye of a patient suffering from pollinosis has mast cells having an IgE antibody to pollen. This IgE antibody captures the antigen component of pollen and binds to it, whereby the mast cell becomes active. As a result, released histamine causes itching through nerves on the surface of the conjunctiva and increases the secretion of tears automatically, and foreign-body sensation is intensified by the hyperactivity of the nerves. Pruritus may be worsened by "scratching". The first choice for the treatment of this symptom is an antiallergenic agent such as chemical mediator isolation inhibitor or histamine H₁ receptor antagonist.

It is known that D-3-hydroxybutyric acid used as an effective component in the present invention is a biogenic substance, formed by oxidizing fatty acid in the liver and used as an energy source in peripheral tissues (Lehninger's New Chemistry (upper volume) under the editorship of Ikuo Yamashina, second edition, Hirokawa Shoten, p.625-626, 1993). It is revealed that D-3-hydroxybutyric acid is used before long-chain fatty acid and glucose and considered as an excellent energy matrix. Making use of this property of D-3-hydroxybutyric acid, a technology for using D-3-hydroxybutyric acid as an infusion compounding ingredient for nutritional support to a patient who suffers from a bio-protein catabolic action increased state or invasion into the body (JP-A 2-191212), a technology for using it as a medicinal composition for the protection of the metabolism of the cardiac muscle (JP-A 58-201746) and a technology for using it as a brain function improving agent (JP-A 10-95730) have been reported. However, in these documents, the antiallergic action of a composition comprising hydroxybutyric acid and/or a salt thereof as an effective component is not studied at all.

Up till now, the applicant of the present application has proposed an intraocular infusion solution (JP-A 10-226704), an agent for the treatment of a corneal epithelial damage (JP-A 10-265378), a composition for the prevention or treatment of an eye trouble caused by apotosis (JP-A 2003-313123), an agent for the treatment of tear abnormality (WO2005/032534A1) and an ophthalmic composition for the suppression of the formation of inflammatory cytokine (JP-A 2005-247821) as a composition comprising 3-hydroxybutyric acid and/or a salt thereof. Out of these, JP-A 2005-247821 shows allergic conjuctivitis as a disorder caused by inflammatory cytokine. However, it is not described that the suppression of inflammatory cytokine eases allergic symptoms, for example, pruritus, and specific examples are not given.

### DISCLOSURE OF THE INVENTION

As described above, 3-hydroxybutyric acid is a biogenic substance and has various pharmacological effects as a medicament. It is therefore an object of the present invention to verify the antiallergenic effect of 3-hydroxybutyric acid and to provide a safe antiallergenic composition.

The inventors of the present invention have conducted intensive studies and have found that sodium D-3-hydroxybutyrate has an excellent pruritus suppression effect on a rat model suffering from chronically allergic conjunctivitis and a rat administered with an ophthalmic solution of an itching substance (histamine).

That is, according to the present invention, the above object of the present invention is attained by an antiallergenic composition which comprises 3-hydroxybutyric acid and/or a salt thereof.

The above 3-hydroxybutyric acid is preferably a D-form.

The above composition is preferably used for an allergy developed in the eye and prepared as an ophthalmic solution or an ophthalmic ointment.

Further, the symptom of the above allergy is preferably pruritus.

### BRIEF DESCRIPTION OF THE DRAWINGS

Fig. 1 shows the relationship between the administration of an HBA ophthalmic solution and the number of times of itching in a rat model suffering from chronically allergic conjunctivitis;
Fig. 2 shows the relationship between the total number of times of itching and the concentration of the HBA ophthalmic solution during 8 days of the administration of the ophthalmic solution in the relationship shown in Fig. 1;
Fig. 3 shows the relationship between the number of eosinophils invaded into the conjunctiva and the concentration of the HBA ophthalmic solution;
Fig. 4 shows the effect of reducing the number of times of scratching by each medicament;
Fig. 5 shows the relationship between the number of eosinophils invaded into the conjunctiva and each medicament;
Fig. 6 shows the suppression of the symptom (hyperemia) of conjunctivitis by each medicament;
Fig. 7 shows the suppression of the symptom (edema) of conjunctivitis by each medicament;
Fig. 8 shows the suppression of the permeability of the conjunctival capillary by each medicament; and
Fig. 9 shows the each medicament's function of suppressing the degranulation of a mast cell.

### BEST MODE FOR CARRYING OUT THE INVENTION

In the present invention, as for the steric configuration of the hydroxyl groups of 3-hydroxybutyric acid as an effective component, there are known D-form, D, L-racemic form and L-form. In the present invention, all of them may be used. As a salt of 3-hydroxybutyric acid, at least one is suitably selected from the group consisting of a sodium salt, potassium salt, L-lysine salt, L-histidine salt and L-arginine salt. These 3-hydroxybutyric acids and/or salts thereof may be suitably used alone or in combination of two or more. The concentration of 3-hydroxybutyric acid and/or a salt thereof in the ophthalmic solution of the present invention which depends on the age and symptom of a patient is preferably 0.01 to 10.0 w/v%, more preferably 0.05 to 5.0 w/v%, particularly preferably 0.1 to 3.0 w/v%.

The composition of the present invention can be formulated by adding additives which are allowed as medicines as required.

The composition of the present invention can be administered non-orally or orally. The oral preparations include liquid preparations for oral administration (such as elixir and syrup) and solid preparations for oral administration (such as tablets, capsules, granules and trochiscis). The non-oral preparations include injectable solutions, ophthalmic solutions, external preparations (such as ointments including eye ointments, creams and adhesive preparations), nasal preparations and inhalants. These preparations may be manufactured by methods described in Japanese Pharmacopoeia.

The formulation of the composition for allergy developed in the eye is preferably an ophthalmic solution, eye ointment or tablet, more preferably an ophthalmic solution. The ophthalmic solution may be mixed with additives such as a tonicity agent, buffering agent, stabilizer, viscosity inducing agent, pH control agent and preservation agent, and other components.

The above tonicity agent may be used to control the osmotic pressure of the ophthalmic solution. Any tonicity agent which is generally used in an ophthalmic solution may be used without a problem, as exemplified by inorganic salts such as alkali or alkali earth metal salts including sodium chloride, potassium chloride, calcium chloride, magnesium chloride and magnesium sulfate and carbohydrates such as glucose, mannitol, sorbitol, xylitol, dextran and glycerin. They may be used alone or in combination of two or more. The concentration of the tonicity agent is preferably 0.001 to 5 w/v%, more preferably 0.01 to 3.0 w/v%.

The above buffering agent may be used to stabilize the pH of the ophthalmic solution. Any buffering agent which is generally used in an ophthalmic solution may be used without a problem, as exemplified by boric acid, citric acid, phosphoric acid, tartaric acid, gluconic acid, acetic acid, carbonic acid and salts thereof. They may be used alone or in combination of two or more. The concentration of the buffering agent is preferably 0.001 to 5 w/v%, more preferably 0.01 to 1 w/v%.

The above stabilizer may be used to stabilize the effective component of the ophthalmic solution. Any stabilizer which is generally used in an ophthalmic solution may be used without a problem, as exemplified by sodium edetate, cyclodextrin, sulfites, citric acid/salt and dibutylhydroxy toluene. They may be used alone or in combination of two or more. The concentration of the stabilizer is preferably 0.001 to 5 w/v%, more preferably 0.01 to 1 w/v%.

The above viscosity inducing agent may be used to control the viscosity of the ophthalmic solution. Any viscosity inducing agent which is generally used in an ophthalmic solution may be used without a problem, as exemplified by polyols such as glycerin, ethylene glycol, propylene glycol, polyethylene glycol and polyvinyl alcohol, carbohydrates such as trehalose, sucrose, carboxymethyl cellulose, hydroxyethyl cellulose, hydroxypropylmethyl cellulose and cyclodextrin, polycarboxylic acids/salts such as carboxyvinyl polymer and citrates and edetates, polysaccharides such as xanthan gum, Locust beam gum, gellan gum and carrageenan, hyaluronic acid/salt, pobidon and castor oil. They may be used alone or in combination of two or more. The concentration of the viscosity inducing agent is preferably 0.001 to 10 w/v%, more preferably 0.01 to 5 w/v%.

The above pH control agent may be used to control the pH of the ophthalmic solution. Any pH control agent which is generally used in an ophthalmic solution may be used without a problem, as exemplified by sodium hydroxide, potassium hydroxide, sodium carbonate, sodium hydrogen carbonate, hydrochloric acid, citric acid, boric acid, phosphoric acid, acetic acid, tartaric acid and salts thereof. They may be used alone or in combination of two or more. A suitable amount of the pH control agent is added to the ophthalmic composition of the present invention to control its pH to the target value.

The above preservation agent may be used to provide a storage effect to the ophthalmic solution. Any preservation agent which is generally used in an ophthalmic solution may be used without a problem, as exemplified by quaternary ammoniums such as benzalkonium chloride, benzethonium chloride and polyquaternium, biguanides such as chlorhexidine gluconate and polyhexamethylene biguanide, benzoates such as methyl p-hydroxybenzoate, chlorobutanol, sorbic acid and potassium sorbate. They may be used alone or in combination of two or more. The concentration of the preservation agent is preferably 0.0001 to 0.1 w/v%, more preferably 0.001 to 0.05 w/v%.

The above other components may be used to provide effects corresponding to these components. Any other components which are generally used in an ophthalmic preparation may be used without a problem, as exemplified by decongestant, anti-inflammation/adstriction agent, regional anesthetic, vitamins, amino acids and refreshing agent. They may be used alone or in combination of two or more. When these components are contained, they are preferably used according to the age and symptom of a patient after it is confirmed that their components and concentrations have no influence upon the antiallergenic effect of 3-hydroxybutyric acid and/or a salt thereof which are/is the effective component (s) of the present invention.

When the composition of the present invention is prepared as an ophthalmic solution, after these components are combined together, the pH of the composition is preferably controlled. The range of pH is not particularly limited if it is allowable as an ophthalmic solution, for example, preferably 4 to 10, more preferably 6 to 8.5. When it is an acidic range at less than 4 or an alkaline range at more than 10, it is possible to cause eye irritancy or eye trouble disadvantageously.

When the composition of the present invention is prepared as an ophthalmic solution, the osmotic pressure of the composition is not particularly limited if it is allowable as an ophthalmic solution but preferably 100 to 600 mOsm., more preferably 150 to 500 mOsm.

When the composition of the present invention is prepared as an ophthalmic solution, its given dosage is not particularly limited if it is allowable ophthalmologically. For example, when the composition is used as an ophthalmic solution, normally one to three drops per time are preferably administered 1 to 20 times a day, particularly preferably 1 to 10 times a day.

As described above, according to the present invention, there is a useful proposal for using 3-hydroxybutyric acid and/or a salt thereof as an antiallergenic agent. This proposal is realized by containing 3-hydroxybutyric acid and/or a salt thereof as an antiallergenic agent in an antiallergenic composition.

### Examples

The following examples are provided for the purpose of further illustrating the present invention but are in no way to be taken as limiting.

### Example 1

The effect of suppressing itching and the effect of suppressing the invasion of eosinophils into the conjunctiva of the composition of the present invention which comprises D-3-hydroxybutyric acid as an effective component were investigated by using a rat model suffering from chronically allergenic conjunctivitis.

### Method of preparing test formulation

Test formulations No. 1 to No. 5 were prepared by dissolving components shown in Table 1 in purified water, controlling the pH of the resulting solution to 7.0 to 7.5 with an aqueous solution of diluted hydrochloric acid or diluted sodium hydroxide and filtering the solution aseptically.

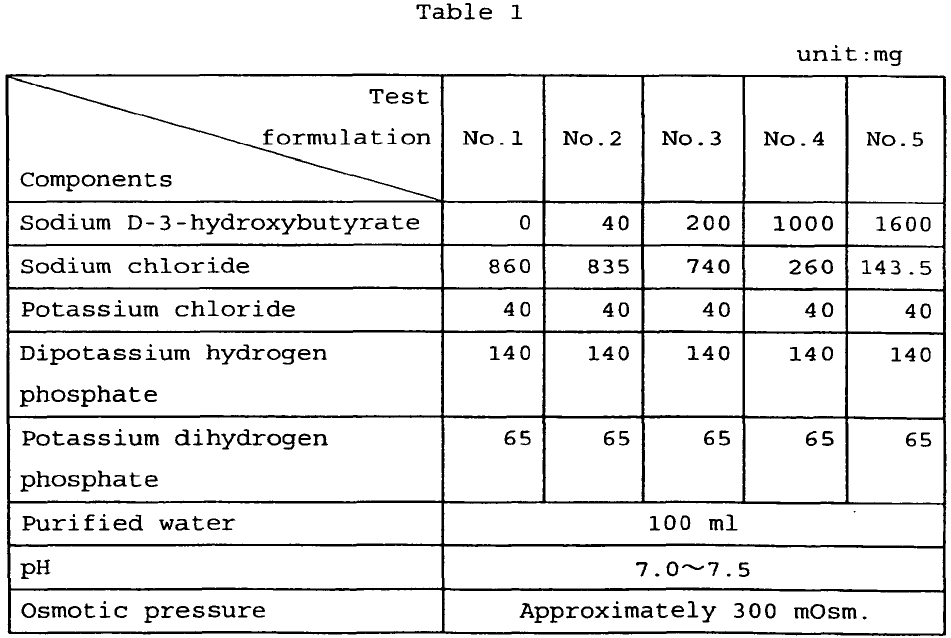

### Preparation of rat model suffering from chronically allergic conjunctivitis

0.6 ml of a primary sensitizing reagent (a solution prepared by dissolving 20 mg of egg albumin, 40 mg of aluminum hydroxide gel and 2 x 10¹¹ units of an inactivated pertussis antigen in physiological saline) was administered to the footpads of both hands and both feet of a rat by using a 24G injection needle. Five days after the administration of the primary sensitizing reagent, 1 ml of a secondary sensitizing reagent (a solution prepared by dissolving 50 mg of egg albumin in 100 ml of physiological saline) was injected hypodermically into the back portion of the rat by using a 27G injection needle. From 14-th to 42-th days after the administration of the primary sensitizing reagent, 5 µl of drops of a local sensitizing reagent (a solution prepared by dissolving 500 mg of egg albumin in 50 ml of physiological saline) was administered to the both eyes of the rat, and the rat was put in an observation cage to observe the "scratching" of the rat for 20 minutes right after the administration of the ophthalmic solution. The rat which took this action 10 times or more within 20 minutes was used as a "rat suffering from experimental allergenic conjunctivitis" in the experiment. The observation of "scratching" was carried out every day after the administration of the drops of the local sensitizing reagent, and a test on the evaluation of an itching suppression effect was carried out after all the sensitized rats got experimental allergenic conjunctivitis. The test on the evaluation of the itching suppression effect was carried out before the 42-nd day after the administration of the primary sensitizing reagent.

### Evaluation test of itching suppression effect

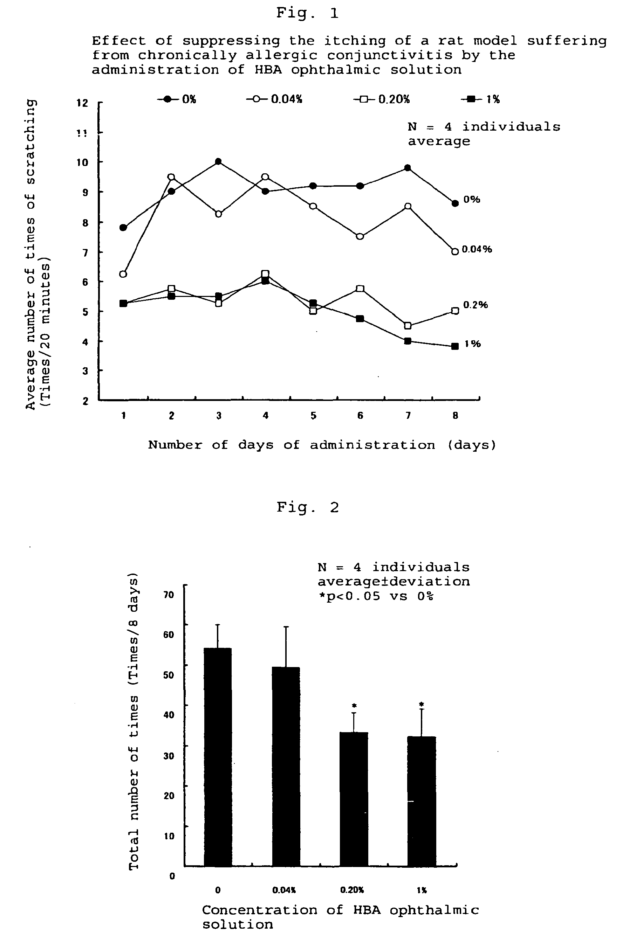

5 minutes after 5 µl of drops of a test formulation was administered to the both eyes of a rat suffering from an experimental allergy, 5 µl of drops of a local sensitizing reagent was administered to these eyes. After the administration of the drops of the local sensitizing reagent, the scratching of the rat was observed for 20 minutes. This operation was repeated for 8 days. The number of times of scratching each day is shown in Fig. 1 and the total number of times of scratching for 8 days is shown in Fig. 2. Sodium D-3-hydroxybutyrate suppressed the scratching of the rat suffering from an experimental allergy, dependent upon its volume, and test formulations No.3 and No. 4 having a sodium D-3-hydroxybutyrate concentration of not less than 0.2 w/v% is superior to test formulation No.1 (Fig. 2, significant level of 5 %, t detection). It was considered from this result that sodium D-3-hydroxybutyrate has the effect of inhibiting the immediate phase of an allergic inflammation reaction.

### Effect of suppressing the invasion of eosinophils into conjunctiva

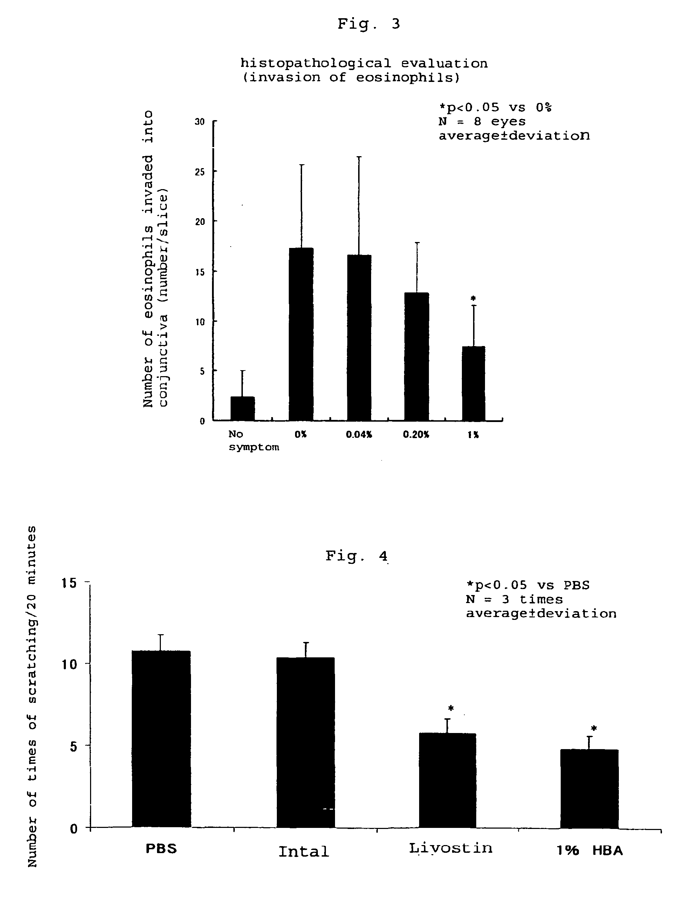

After "the evaluation test of itching suppression effect", an eyeball was extracted from the rat and washed with PBS. The eyeball was halved at around the periphery of an optic nerve region and its keratoconjunctiva side was fixed with a formalin solution at room temperature for one night. The conjunctiva was cut out from the fixed eyeball, washed with a phosphoric acid buffer solution having a concentration of 0.1 mol/l and immersed in ethanol. Thereafter, it was immersed in xylene and paraffin 3 times each and embedded in paraffin in the end. The embedded tissue was sliced to a thickness of 4 µm, dried for 2 days and stained light giemsa in the end. The slice prepared by this method was used as a tissue sample and observed through a microscope. When the number of invaded eosinophils for each sample was counted, test formulation No. 4 having a sodium D-3-hydroxybutyrate concentration of 1.0 w/v% suppressed the invasion of eosinophils into the conjunctival tissue more significantly than test formulation No.1 (Fig. 3, significant level of 5 %, t detection). It was considered from this result that sodium D-3-hydroxybutyrate has the effect of inhibiting the late phase of an allergic inflammation reaction.

### Example 2

The effect of suppressing the pruritus of the eyes of a rat caused by the administration of an ophthalmic solution of histamine and the effect of suppressing the invasion of eosinophils of the composition comprising 3-hydroxybutyric acid as an effective component of the present invention were investigated.

### Test formulations

In experiments on the evaluation of the effect of suppressing histamine-induced pruritus, test formulation No. 1 (0 % of sodium D-3-hydroxybutyrate, referred to as PBS in Fig. 4) and test formulation No. 4 (1.0 % of sodium D-3-hydroxybutyrate, referred to as 1 % HBA in Fig. 4) shown in Table 1 and Intal (2 % of sodium cromoglycate) and Livostin (0.025 % of levocabastine hydrochloride) as Comparative formulation were used.

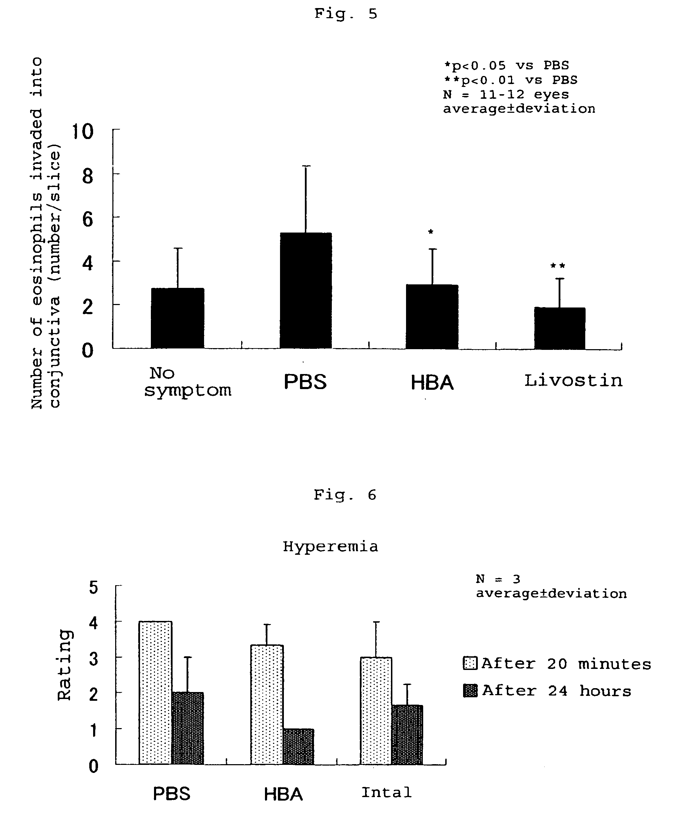

In experiments on the evaluation of the effect of suppressing the invasion of eosinophils into the conjunctiva, test formulation No.1 (0 % of sodium D-3-hydroxybutyrate, referred to as PBS in Fig. 5) and test formulation No. 4 (1.0 % of sodium D-3-hydroxybutyrate, referred to as HBA in Fig. 5) shown in Table 1 and Livostin (0.025 % of levocabastine hydrochloride) as a Comparative formulation were used.

### Effect of suppressing histamine-induced pruritus

5 µl of an ophthalmic solution of the test formulation or Comparative formulation was administered to the both eyes of each rat each time. Five minutes after this, 10 µmol/5 µl of an ophthalmic solution of histamine was administered to one eye each time to induce pruritus in the rat. When "scratching" was observed for 20 minutes right after the administration of the histamine ophthalmic solution, test formulation No. 4 (1 w/v% of sodium D-3-hydroxybutyrate) showed the same level of the effect of suppressing itching as that of Livostin which is a histamine H₁ receptor competitive inhibitor. Meanwhile, Intal which is a chemical mediator isolation inhibitor was not effective (Fig. 4). It is suggested from this result that sodium D-3-hydroxybutyrate has antihistaminic action.

### Effect of suppressing the invasion of eosinophils into conjunctiva

5 µl of an ophthalmic solution of the test formulation or Comparative Example was administered to the both eyes of each rat each time. Five minutes after this, 10 µmol/5 µl of an ophthalmic solution of histamine was administered to one eye each time to induce pruritus in the rat. 24 hours after the administration of the ophthalmic solution of histamine, an eyeball was extracted from the rat and washed with PBS. After washing, the periphery of the optic nerve region of the rat was cut and the eyeball was fixed with a formalin solution at room temperature for one night. The conjunctiva was cut out from the fixed eyeball, washed with a phosphoric acid buffer solution having a concentration of 0.1 mol/l and immersed in ethanol. Thereafter, it was immersed in xylene and paraffin 3 times each and embedded in paraffin in the end. The embedded tissue was sliced to a thickness of 4 µm, dried for 2 days and stained light giemsa in the end. The slice prepared by this method was used as a tissue sample and observed through a microscope. When the number of invaded eosinophils in each sample was counted, test formulation No. 4 having a sodium D-3-hydroxybutyrate concentration of 1.0 w/v% suppressed the invasion of eosinophils into the conjunctival tissue more significantly than test formulation No. 1 (Fig. 5, significant level of 5 %, Dunnett test). The number of eosinophils invaded into conjunctiva of the rats administered with the ophthalmic solution of Livostin was also significantly reduced as compared with that of the rats administered with the ophthalmic solution of PBS (Fig. 5, significant level of 1 %, Dunnett test). It was considered from this result that sodium D-3-hydroxybutyrate has the effect of inhibiting the late phase of an allergic inflammation reaction.

### Example 3

The effect of suppressing the symptom of conjunctivitis using a Compound 48/80 induced conjunctivitis rat model, the effect of suppressing the increase of vascular permeability and the effect of suppressing the invasion of eosinophils of the composition comprising 3-hydroxybutyric acid as an effective component of the present invention were investigated.

### Test formulations

In experiments on the evaluation of the effect of suppressing the symptom of conjunctivitis and the effect of suppressing the invasion of eosinophils, test formulation No. 1 (referred to as PBS in Figs. 6, 7, 8 and 9) and test formulation No. 5 (referred to as HBA in Figs. 6, 7, 8 and 9) shown in Table 1 and Intal (2 % of sodium cromoglycate) as a Comparative formulation were used.

In experiments on the evaluation of the effect of increasing vascular permeability, Livostin (0.025 % of levocabastine hydrochloride) was used as a Comparative formulation.

### Effect of suppressing the symptom of conjunctivitis

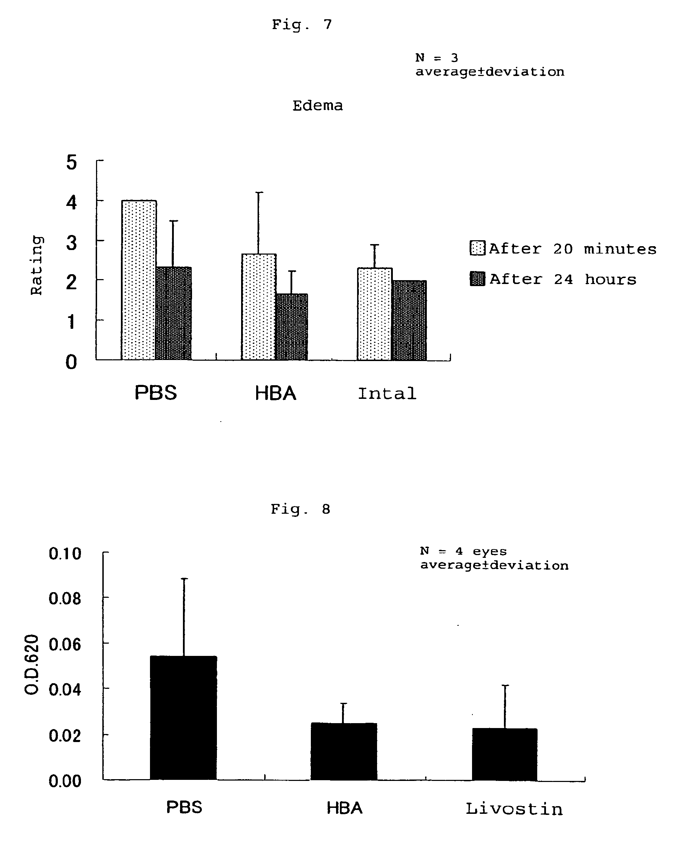

5 µl of an ophthalmic solution of the test formulation or Comparative formulation was administered to the both eyes of each rat each time. Five minutes after this, 2 mg/5 µl of an ophthalmic solution of Compound 48/80 was administered to one eye each time to develop conjunctivitis in the rat. 20 minutes and 24 hours after the administration of the ophthalmic solution of Compound 48/80, the symptom of conjunctivitis was evaluated. Hyperemia and edema were evaluated by rating shown in Table 2. As a result, the ratings of the rats administered with the ophthalmic solution of HBA were lower than those of the rats administered with the ophthalmic solution of PBS in terms of edema after 20 minutes and hyperemia after 24 hours. In the rats administered with an ophthalmic solution of Intal which is a chemical mediator isolation inhibitor, edema and hyperemia after 20 minutes tended to be suppressed more than those of the rats administered with the ophthalmic solution of PBS (Figs. 6 and 7).

**Table 2**

| Rating | Symptoms | |
|---|---|---|
| | Hyperemia | Edema |
| 0 | No symptom | No symptom |
| 1 | Slight hyperemia in one eye | Slight edema in one eye |
| 2 | Slight hyperemia in both eyes | Slight edema in both eyes |
| 3 | Marked hyperemia in one eye and slight hyperemia in the other eye | Marked edema in one eye and slight edema in the other eye |
| 4 | Marked hyperemia in both eyes | Marked edema in both eyes |

Compound 48/80 promotes the degranulation of mast cells to induce the symptoms of conjunctivitis such as hyperemia and edema. It is considered that Intal which is a chemical mediator isolation inhibitor suppresses the degranulation of mast cells to suppress the symptoms of conjunctivitis at the time of administering the ophthalmic solution of Compound 48/80. It is suggested that HBA may also suppress the degranulation of mast cells to suppress the symptoms of conjunctivitis.

### Effect of increasing vascular permeability

A 2 % Evans blue solution was administered to the rat administered with the test formulation No.1 or No.5 or Comparative formulation. Each administration was conducted by injecting into the vein of the tail. Five minutes after the administration, 2 mg/5 µl of the ophthalmic solution of compound 48/80 was administered to one eye each time to develop conjunctivitis in the rat. 20 minutes after the administration of the ophthalmic solution of compound 48/80, the rat was exsanguinated to death to sample its conjunctiva. The sampled conjunctiva was put into a mixed solution of acetone and sodium sulfate, and a pigment leaked out into the conjunctiva was extracted. After extraction, the 620 nm light absorbance of the pigment was measured to determine and evaluate the amount of the pigment leaked out into the conjunctiva. As a result, the amount of the leaked pigment in the rats administered with HBA became smaller than that in the rats administered with PBS. The amount of the pigment in the rats administered with Livostin became smaller than that in the rats administered with PBS (Fig. 8).

It is known that a chemical mediator such as histamine released from the mast cells degranulated by compound 48/80 increases the capillary permeability of the conjunctiva. It is suggested that HBA may suppress an increase in capillary permeability by the administration of compound 48/80 through competition with histamine.

### Effect of suppressing the invasion of eosinophils into conjunctiva

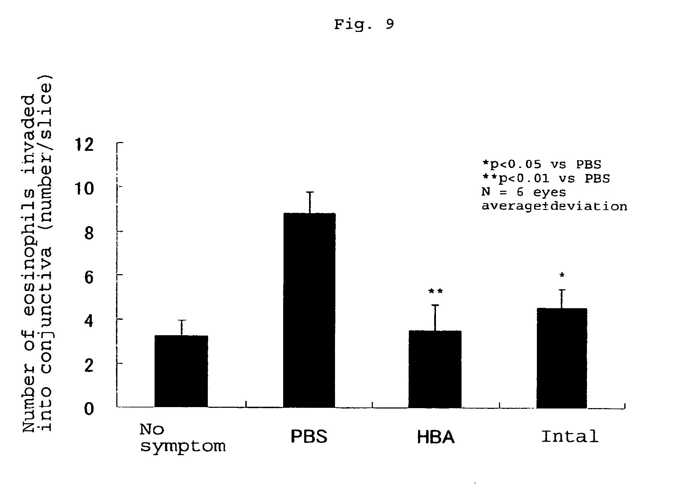

5 µl of an ophthalmic solution of the test formulation or Comparative formulation was administered to the both eyes of each rat each time. Five minutes after this, 2 mg/5 µl of an ophthalmic solution of compound 48/80 was administered to one eye each time to develop conjunctivitis in the rat. 24 hours after the administration of the ophthalmic solution of compound 48/80, an eyeball was extracted from the rat and washed with PBS. The eyeball was halved at around the periphery of an optic nerve region, and its keratoconjunctiva side was fixed with a formalin solution at room temperature for one night. The conjunctiva was cut out from the fixed eyeball, washed with a phosphoric acid buffer solution having a concentration of 0.1 mol/l and immersed in ethanol. Thereafter, it was immersed in xylene and paraffin 3 times each and embedded in paraffin in the end. The embedded tissue was sliced to a thickness of 4 µm, dried for 2 days and stained light giemsa in the end. The slice prepared by this method was used as a tissue sample and observed through a microscope. The number of eosinophils invaded into the conjunctivas of the rats administered with HBA was significantly reduced as compared with that of the rats administered with PBS (Fig. 9, significant level of 1 %, Dunnett test). The number of eosinophils invaded into the conjunctivas of the rats administered with Intal was significantly reduced as compared with that of the rats administered with PBS (Fig. 9, significant level of 5 %, Dunnett test).

An eosinophil invasion induction substance such as an eosinophil chemotactic factor is released from mast cells degranulated by compound 48/80. It is considered that Intal which is a chemical mediator isolation inhibitor suppresses degranulation by the film stabilization effect of the mast cells with the result that the invasion of eosinophils is suppressed. It is suggested that HBA may also have the effect of suppressing the degranulation of the mast cells.

As described above, the composition of the present invention can suppress a symptom caused by an allergic reaction, for example, pruritus. It is particularly effective for itching which occurs in the eye. Since hydroxybutyric acid and a salt thereof which are effective components of the composition are soluble in water and stable in an aqueous solution, they are suitable for use as an ophthalmic solution (including an eye wash and contact lens wearing solution) applied to the eyes.

## Claims

1. A composition for use in a method of treating allergy, the composition comprising 3-hydroxybutyric acid and/or a salt thereof.

2. The composition for use according to claim 1, wherein the hydroxybutyric acid is a D-form.

3. The composition for use according to claim 2, wherein the allergy occurs in an eye.

4. The composition for use according to claim 3, wherein the symptom of the allergy is pruritus.

5. The composition for use according to claim 4 which is prepared as an ophthalmic solution or ophthalmic ointment.

6. 3-hydroxybutyric acid and/or a salt thereof for use as an antiallergenic agent.

## Patentansprüche

1. Zusammensetzung zur Verwendung in einem Verfahren zur Behandlung von Allergie, wobei die Zusammensetzung 3-Hydroxybuttersäure und/oder ein Salz davon umfasst.

2. Zusammensetzung zur Verwendung gemäß Anspruch 1, wobei die Hydroxybuttersäure die D-Form ist.

3. Zusammensetzung zur Verwendung gemäß Anspruch 2, wobei die Allergie in einem Auge auftritt.

4. Zusammensetzung zur Verwendung gemäß Anspruch 3, wobei das Symptom der Allergie Pruritus ist.

5. Zusammensetzung zur Verwendung gemäß Anspruch 4, die als ophthalmische Lösung oder ophthalmische Salbe hergestellt ist.

6. 3-Hydroxybuttersäure und/oder ein Salz davon zur Verwendung als Antiallergikum.

## Revendications

1. Composition pour l'utilisation dans un procédé de traitement de l'allergie, la composition comprenant l'acide hydroxybutyrique et/ou un sel de celui-ci.

2. Composition pour l'utilisation selon la revendication 1, dans laquelle l'acide hydroxybutyrique est la forme D.

3. Composition pour l'utilisation selon la revendication 2, l'allergie se produisant dans un oeil.

4. Composition pour l'utilisation selon la revendication 3, le symptôme de l'allergie étant le prurit.

5. Composition pour l'utilisation selon la revendication 4 qui est préparée comme une solution ophtalmique ou une pommade ophtalmique.

6. Acide hydroxybutyrique et/ou un sel de celui-ci pour l'utilisation en tant qu'agent antiallergique.
